# EUROPEAN PATENT APPLICATION

(11) **EP 4 400 574 A1**
(43) Date of publication of application: **17.07.2024**
(21) Application number: 23151430.8
(22) Date of filing: 12.01.2023
(51) Int. Cl.: C12M 1/107, C12M 1/24, C12M 1/12, C12P 5/00

(54) **A SYSTEM AND A METHOD FOR PRODUCTION OF VOLATILE NATURAL COMPOUNDS USING SOLID GROWTH MEDIA**

(71) Applicant: Univerza v Mariboru, 2000 Maribor (SI)
(72) Inventor: Zebec, Ziga, 2000 Maribor (SI); Lobnik, Aleksandra, 2000 Maribor (SI)
(74) Representative: Patentni Biro AF d.o.o.

(57) **Abstract**

The invention relates to a system and a method for production of volatile natural compounds using microorganisms grown on solid growth media. The system comprises a flask with one open end arranged to receive a solid growth medium and a closure arranged to fit in said open end of the flask, said closure being configured to receive two separated, different liquids. The container allows sealing in order to prevent the produced VOCs from escaping the container. The method comprises at least the following steps:
a) preparation of the container and a suitable solid growth medium,
b) growth of a selected microbial strain,
c) inoculation of the microbial strain onto the solid growth medium,
d) filling the closure with water and a suitable organic solvent separately,
e) sealing, and
f) incubation, wherein VOCs are present in the organic solvent.

## Description

### Field of the invention

The present invention belongs to the field of methods for the preparation of compounds or compositions using microorganisms or enzymes, particularly to the field of methods for the preparation of volatile compounds. The invention relates to a system and a method for production of volatile natural compounds using microorganisms grown on solid growth media.

### Background of the invention and the technical problem

Volatile organic compounds (VOCs) are typically small, odorous compounds (<C₁₅) with low molecular mass (<300 Da), high vapor pressure, low boiling point, and a lipophilic moiety. High vapor pressure correlates with a low boiling point, which relates to the number of VOC molecules in the surrounding air. VOCs are responsible for the odour of scents and perfumes as well as pollutants. Further, VOCs play an important role in communication between animals and plants, e.g., attractants for pollinators, protection from predation, and even inter-plant interactions.

Many VOCs are used and produced in the manufacture of various goods, such as foods, pharmaceuticals, cosmetics, household products, paints, and refrigerants. Limonene, as an example of VOCs, is used to promote weight loss, prevent cancer, treat cancer, and treat bronchitis. In foods, beverages, and chewing gum, limonene is used as a flavouring. In pharmaceuticals, limonene is added to help medicinal ointments and creams penetrate the skin.

When produced in usual settings, the amount of obtained VOCs is limited and needs concentrating. It would be desirable if particularly useful VOCs were produced in substantially higher amounts or concentrations, respectively. Thus, it is an object of the present invention to provide a system and a method that allows producing VOCs in higher amounts and in higher purity.

### Prior art

Documents WO2007060233, WO2007060235 and EP1957656B1 disclose classical methods of producing VOC in liquid media.

Patent application EP2850197A1 discloses a method for processing a biomass material comprising:
- contacting a solid component of a biomass material with a solution adapted to facilitate saccharification, and
- contacting the at least one fermentable sugar with a microorganism capable of using the at least one fermentable sugar to generate a hydrocarbon.
- wherein the solid component is generated by a method comprising:
- introducing a biomass material to a compartment of a solventless recovery system, wherein the biomass material contains one or more volatile organic compounds; contacting the biomass material with a superheated vapor stream in the compartment to vaporize at least a portion of an initial liquid content in the biomass material, said superheated vapor stream comprising at least one volatile organic compound;
- separating a vapor component and a solid component from the heated biomass material, said vapor component comprising at least one volatile organic compound;
- retaining at least a portion of the gas component for use as part of the superheated vapor stream;
- discharging the solid component from the solventless recovery system.
This differs from the present invention.

Patent EP2850199B1 discloses use of biomass material in a method for producing at least one volatile organic compound, said method comprising the following steps:
- generating at least 10 tons of prepared biomass material comprising at least one additive added to a solid biomass comprising a sugar, wherein said at least one additive comprise a microbe, and optionally, an acid and/or an enzyme;
- storing the prepared biomass material for at least 24 hours in a storage facility to allow for the production of at least one volatile organic compound from at least a portion of the sugar; and
- capturing the at least one volatile organic compound by feeding the stored prepared biomass material to a solventless recovery system to separate the stored prepared biomass material into at least a vapor component comprising the at least one volatile organic compound and a solid component, wherein the capturing step comprises:
- introducing the stored prepared biomass material to a compartment of the solventless recovery system;
- contacting the stored prepared biomass material with a superheated vapor stream in the compartment to vaporize at least a portion of an initial liquid content in the stored prepared biomass material, the initial liquid content comprising from 2 wt% to 50 wt% ethanol based on the initial liquid content, and said superheated vapor stream comprising at least one volatile organic compound, wherein the superheated vapor is a vapor that is heated above its saturation temperature at the pressure of operation;
- separating a vapor component and a solid component from the prepared biomass material;
- retaining at least a portion of the vapor component for use as part of the superheated vapor stream; and
- collecting the vapor component, wherein the collected vapor component contains between 4 wt% and 15 wt% ethanol.

Zebec et al (2022; doi: 10.3390/life12091423) used the following protocol for production and isolation of limonene, wherein the growth media were liquid and prepared from various waste materials. *E. coli* cells transformed with plasmid pJBEI-6410 were used for the production of limonene. Three individual colonies were grown in 300 µL of non-selective LB medium in a 1.5 mL plastic tube for 3 h. After 3 h, 40 µL of the cell culture was used to inoculate 5 mL of M9 or LB media supplemented with the appropriate glucose juice as a carbon source. The final glucose concentration was set to 0.4%. To increase the cell density of the 5 mL cell culture, it was grown at 30 °C and 200 rpm for 3 h. The production of limonene was induced by the addition of 25 µM isopropyl-β-D-thiogalactoside (IPTG), while limonene was entrapped in 10% (v/v) organic overlay (dodecane). Cells were grown at 30 °C and 200 rpm for 72 h before the organic overlay was extracted and the optical density (OD) of the cell culture was measured. The extracted overlay was separated by centrifugation for 10 min at 15,000 rpm at 4 °C and dried with MgSO₄ to remove any water. Another centrifugation step was added for 5 min at 4 °C and 15,000 rpm to sediment the MgSO₄. Finally, the dried overlay was decanted and an equal volume of ethyl acetate containing 0.1% sec-butylbenzene was added. The later chemical sec-butylbenzene serves as an internal standard to control the injection volume in gas chromatography (GC).

Patent application WO2014165174A2 discloses various fungal strains able to produce 1, 8-cineole and 1-methyl-1,4-cyclohexadiene either in a liquid or solid medium. Similarly, patent application US2011287471A1 describes fungal strains for production of VOCs, particularly 1,8-cineole, 1-methyl-1,4-cyclohexadiene, and (+)-α-methylene-a-fenchocamphorone. None of these documents disclose a system for production of VOC similar to the present invention.

### Description of the solution to the technical problem

The present invention addresses the problem of efficiency of VOC production processes. The technical problem is solved as defined in the independent claims, wherein the preferred embodiments are defined in dependent claims.

The essence of the invention is that a solid growth medium is used to produce VOC using suitably prepared microorganisms grown on said solid growth medium.

In the first aspect, the invention relates to a system (also called container) for production of VOCs. In the second aspect, the invention relates to a method for producing VOCs.

In the first aspect, the system comprises:
- a flask or any other suitable receptacle with an open end and a closed end, which is arranged to receive a solid growth medium,
- at least one collection compartment, which may be shaped as a closure, a vial or any other suitable receptacle arranged to fit in said open end of the flask, said collection compartment being configured to receive produced VOC by organisms grown in the flask, and
- preferably sealing means installed between the flask and the collection compartment and/or at the junction of the flask and the collection compartment for preventing produced VOCs from escaping from the system.
Said system is made from a suitable material that is inert and allows sterilization according to standard microbiological procedures. The container also has to allow suitable sealing in order to prevent the produced VOCs from escaping the system. Said sealing means may be a seal (gasket), a sealing tape, a sealing device or any other suitable component preventing VOCs from leaving the system.

In a possible embodiment the collection compartment may be empty or may be filled with an organic solvent, in which the produced VOC is soluble. The produced VOCs are present on the walls of the collection compartment and/or in the organic solvent, if used.

The collection compartment may be divided into two parts, wherein an inner part is arranged to receive the organic solvent, while the outer part surrounding said inner part is arranged to receive a substance in which the produced VOC is not soluble. The outer part functions as sealing means, as the non-soluble substance prevents the produced VOCs from escaping from the system.

The orientation of the system may be:
- the flask with the growth medium is placed on a surface and the collection compartment is installed above the flask
- the flask with the growth medium is turned upside-down and placed above the collection compartment, which is placed on a surface.

For harvesting of produced VOCs, the system according to the invention is opened and the produced VOCs either condensed on the walls and/or in the collection compartment or in the solvent present in the collection compartment are collected in a fresh container, which may be any suitable container known to the skilled person.

In a possible embodiment of the container, the solid growth medium is prepared according to standard microbiological procedures, poured in the sterile flask and left to solidify. A culture of selected microbial strain is inoculated, preferably sprayed onto the solidified growth medium, then the flask is turned upside down and closed with the collection compartment, which may be designed as an appropriate first vial comprising an organic solvent. The flask closed with the first vial is inserted into a second vial at least partially filled with water, said second vial being larger than the first vial. The second vial seals the flask and allows entrapment of produced VOCs. An additional seal may be installed to seal the system and the junction of vials and/or flask.

The first and second vial may also be designed as one two-compartment vial, said compartments being arranged to receive two different liquids, i.e., one organic solvent and water.

According to a different embodiment, the collection compartment, which may be empty or filled with an organic solvent, is after inoculation placed above the flask and a seal is installed between the flask and the collection compartment.

The growth medium may be any suitable medium for cultivating microbial strain selected for the procedure of producing VOCs. The preferred media are Luria-Bertani (LB), Terrific Broth (TB), minimal growth medium M9 and similar defined mineral media provided with a suitable amount of agar, which is typically between 0.1 and 3%, preferably between 1 and 3% (1% is 1g per 100 mL of liquid). The selected growth medium may be further supplemented with one or more of the following:
- an inducer, which allows induction of VOCs production in case the microbial strain is provided with an inducible promotor for expression of a gene for VOCs synthesis, wherein examples of a suitable inducer are IPTG, arabinose, tetracycline, rhamnose and similar,
- an appropriate antibiotic or a combination of antibiotics, which allow selection of microbial cells that have successfully received a gene for VOCs synthesis,
- pure carbon source, such as glucose, glycerol, etc. or glucose sources from various sources, said glucose sources being untreated or treated, wherein the glucose source may be hydrolysed waste streams (i.e., glucose juice from cellulose containing waste; ethylene glycol from PET containing waste, etc.),
- other optional components of microbial growth media, such as nitrogen sources and other element sources, enzymes, precursor and/or catalysts....

The organic solvents may be any suitable, in which the produced VOC is soluble, and can be selected in the group comprising:
- aromatic hydrocarbon compounds, e.g., benzene and toluene,
- alkanes, e.g., dodecane and nonane,
- alkyls,
- alcohols, e.g., methanol,
- esters and ethers,
- ketones, e.g., acetone,
- amines, amides,
- nitrated and halogenated hydrocarbons,
- DMSO,
wherein preferred choices are dodecane or nonane.

The organic solvents may be used alone or in any possible combination of the above-mentioned possible solvents.

The second liquid is water or any other liquid, in which the produced VOC is not soluble. The second liquid is intended to prevent VOC from escaping from the container. However, it is possible to construct the container without the vial for the second liquid as indicated in the description above. In this case a suitable seal is installed, which also prevents the produced VOC from escaping from the system.

The method for production of volatile natural compounds using microorganisms grown on solid growth media as the second aspect of the invention, comprises at least the following steps:
a) preparation of the above-described container and a suitable solid growth medium for inoculation with the selected microbial strain,
b) growth of the selected microbial strain in suitable growth medium,
c) inoculation of the microbial strain prepared in step b) onto the solid growth medium prepared in the container in step a),
d) filling the closure or the vial of the container with water and a suitable organic solvent separately,
e) sealing of the container using a suitable seal, sealing tape, or any other sealing device,
f) incubation of the container for at least 18 hours, preferably 72 hours, at 20 to 40 °C, preferably at 30 °C,
g) opening of the container, wherein the produced VOC is present in the first vial or the closure of the container.

The microbial strain used in the process according to the invention may be any suitable strain known to naturally produce VOC or genetically engineered to produce VOC either spontaneously or following an induction with a suitable inducer. The preferred microbial strains are strains of genus *Bacillus, Escherichia coli, Saccharomyces cerevisiae,* fungal strains and similar well-known and well-studied strains. The selected strain may be further provided with one or more genes allowing production of VOCs, either by transformation or any other gene insertion method. For this purpose, any suitable gene-carrier may be used. Preferably, transformation of competent cells is carried out with a plasmid comprising suitable heterologous genes for production of natural VOCs. As an example, plasmid pJBEI-6410 for production of limonene in *E*. *coli* may be used. Transformed cells are plated on agar plates with appropriate selective components, preferably antibiotic. Any colony observed on the agar plate may be used in the process as described above, wherein the growth medium is for so prepared cells provided with an inducer or the inducer is added later for triggering gene expression and thus VOC synthesis.

The container and the method according to the invention may be used for the production of different VOCs, for example limonene, various terpenes, such as g-terpinene, fenchol, a-terpineol, sabinene, (E)-b-ocimene, camphene, b-phellandrene, and terpinolene. The VOCs obtained with the container and the method according to the invention exhibit high purity and may be used in production of biofuels, value-added biochemicals and monomers for the production of biological drugs as well as advanced biological materials.

The system and the method according to the invention allow simpler, more efficient, and more cost-efficient production of VOCs, wherein less energy is needed for the process in comparison to known methods that include fractioning.

### Brief description of the drawings

The invention will be further described using examples and figures, which show:
- Figure 1: A system for production of volatile natural compounds according to a first embodiment
- Figure 2: A system for production of volatile natural compounds according to a second embodiment

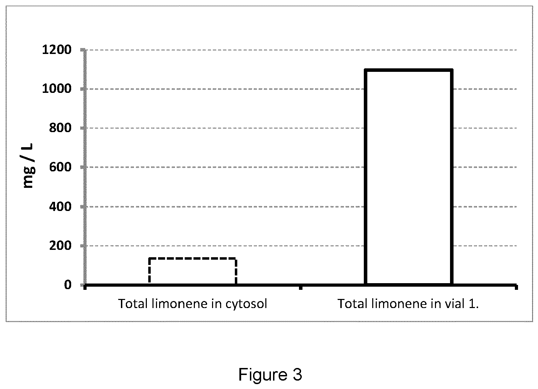

- Figure 3: Produced limonene extracted from all cells (present in cytosol) and produced limonene captured in the system according to the invention

### Detailed description of the invention

The system for production of VOCs according to the invention comprises:
- a flask or any other suitable receptacle 1 with an open end 1a and a closed end 1b, which is arranged to receive a solid growth medium 1c,
- at least one collection compartment 2, which may be shaped as a closure, a vial or any other suitable receptacle arranged to fit in said open end of the flask 1, said collection compartment 2 being configured to receive produced VOC by organisms grown in the flask, and
- preferably sealing means 3 installed between the flask and the collection compartment and/or at the junction of the flask and the collection compartment for preventing produced VOCs from escaping from the system.

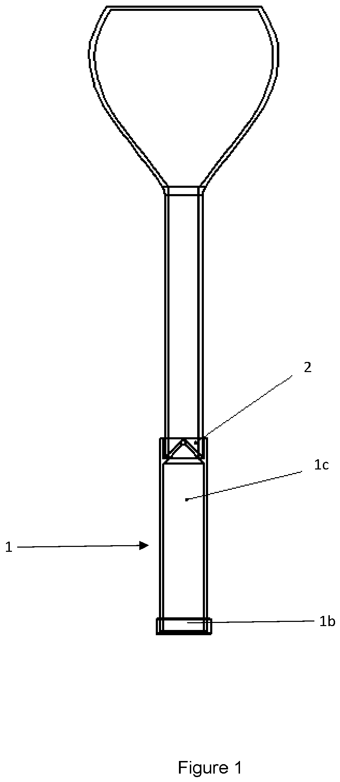

The embodiment of the system shown in figure 1 has the orientation wherein the flask 1 with the growth medium 1c is placed on a surface and the collection compartment 2 is installed above the flask 1.

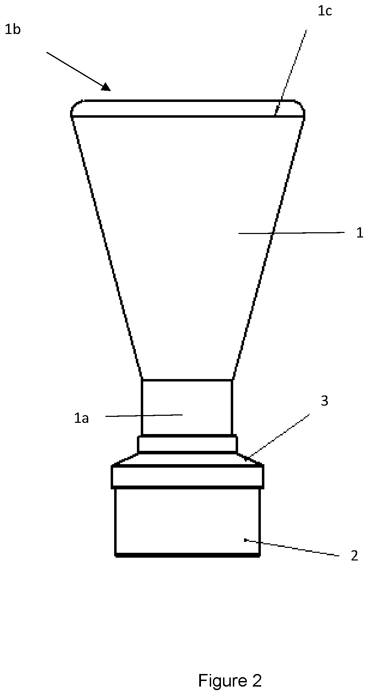

The embodiment of the system shown in figure 2 has the orientation, in which the flask 1 with the growth medium 1c is turned upside-down and placed above the collection compartment 2, which is placed on a surface.

In both embodiments the collection compartment is filled with an organic solvent, in which the produced VOC is soluble. The produced VOCs are present on the walls of the collection compartment and/or in the organic solvent.

Preparation of the system includes preparation of a selected growth medium, such as LB, TB, M9, etc. containing Agar, inducer (IPTG), appropriate antibiotics with ½ concentration and pure carbon source (such as glucose, glycerol, etc.) or glucose source (directly or treated) from hydrolysed waste streams (glucose juice from cellulose containing waste; ethylene glycol from PET containing waste, etc.), which depend on the selected organism for production of VOCs. These particulars are adjusted with regards to the organism, conditions for growth, conditions for VOC production and are obvious to the skilled person.

### Examples

### Example 1: Microbial Strain preparation

The preferred microbial strain for use in the method according to the invention is *E*. *coli* and the preparation comprises genetic manipulation of E. coli cells with heterologous genes allowing production of VOCs, said preparation comprising the following steps:
- Preparation of competent *E*. *coli* cells
- Transformation of competent cells prepared in the first step with the plasmid containing heterologous genes for the production of natural compounds, for example plasmid pJBEI-6410 for production of limonene in *E*. *coli,* and
- Plating the transformed cells on agar plates with appropriate antibiotic for selection of successfully transformed cells.

### Example 2: Preparation of inoculate for production of VOCs

The transformed cells are used in preparation of a suitable inoculate for the method for production of VOCs and the inoculate preparation comprises the following steps:
- Picking at least one colony from agar plate with successfully transformed cells from Example 1
- Inoculation of at least one colony in a suitable liquid media, such as LB, TB, M9, etc., with ½ concentration of the appropriate antibiotic, for example 50µg/ml for Ampicillin in *E*. *coli*;
- Incubation of the inoculated growth media on orbital shaker set at 300rpm for 3h and at temperature of 37 °C; and
- Diluting the culture obtained in previous step to an appropriate OD for induction with IPTG (OD= 0.2 for *E*. *coli*)*.*

### Example 3: Preparation of the container for the production of VOCs and production of VOCs

The preparation of the container comprises:
- preparation of selected growth medium, which was TB comprising 1.5% agar, 0.4% glucose, inducer IPTG, appropriate antibiotics at ½ of the usual concentration,
- allowing the growth medium to solidify in the flask,
- spraying (dispersing) cells with low OD prepared in Example 2 onto the solidified media,
- closing the flask with the collection compartment filled with organic solvent or without solvent,
- sealing the system completely and entrap the produced VOCs, and
- incubation of the sealed system for 72h at 30 °C.

The produced VOCs are present in the organic solvent and/or on the walls of the collection compartment.

### Example 4: Production and quantification of volatile natural compounds

Upon production of VOCs in Example 3, the latter are quantified and identified using the following steps:
- opening of the container,
- drying the sample with MgSO₄, where a small spatula anhydrous MgSO₄ is added and the mixture is vortexed,
- spinning for 3 min. in centrifuge 13000g- 16000g.
- transferring samples and standard to HPLC vial,
- adding 1 volume of ethyl acetate comprising the internal standard, 0.1 % butyl benzene), min., wherein the final volume is 700µl
- preparation of standard, which is 1mg/ml standard in organic solvent (dodecane), if the organic solvent was used, and dilute 1:1 with ethyl acetate comprising the internal standard 0.1 % sec. benzene,
- transferring samples and standard to GC or GC/MS, using DB-WAX or VF-5ms column, and
- analysis of GC or GC/MS to obtain concentrations of individual VOCs produced in Example 3.

Figure 2 shows produced limonene extracted from all cells (present in cytosol) and produced limonene captured in vial or in the organic solvent present in the vial.
For quantification of produced limonene present in the cytosol of cells, all cells growing on the agar were collected in PBS buffer. The cells were lysed by sonication to break the cells and release the cytosol. Limonene was extracted by ethyl acetate and quantified directly on GC/MS. The amount of limonene in cytosol vas 135.4 mg/L.

The amount of limonene in collection compartment as determined in example 4 was 1096.3 mg/L, which is 8-fold higher than the amount of limonene in cytosol. These results suggest that the invention allows production of limonene and VOCs in a significantly higher amount.

## Claims

1. A system for production of volatile natural compounds using microorganisms grown on solid growth media, wherein the system comprises:
- a flask or any other suitable receptacle (1) with an open end (1a) and a closed end (1b), which is arranged to receive a solid growth medium (1c) for growing microorganisms, and
- at least one collection compartment (2), which may be shaped as a closure, a vial or any other suitable receptacle arranged to fit in said open end (1a) of the flask (1), said collection compartment (2) being configured to receive produced VOC by microorganisms grown in the flask (1).

2. The system according to claim 1 further comprising:
- sealing means (3) installed between the flask (1) and the collection compartment (2) and/or at the junction of the flask (1) and the collection compartment (2) for preventing produced VOCs from escaping from the system.

3. The system according to claim 2, wherein said sealing means (3) may be a seal (gasket), a sealing tape, a sealing device or any other suitable component preventing VOCs from leaving the system.

4. The system according to any of the preceding claims, wherein the collection compartment (2) is divided into two parts, wherein an inner part is arranged to receive the organic solvent, while an outer part surrounding said inner part is arranged to receive a substance in which the produced VOC is not soluble or air.

5. The system according to any of the preceding claims, wherein the orientation of the system is that
- the flask (1) with the growth medium (1c) is placed on a surface and the collection compartment (2) is installed above the flask (1), or
- the flask (1) with the growth medium (1c) is turned upside-down and placed above the collection compartment (2), which is placed on a surface.

6. The system according to any of the preceding claims, wherein the collection compartment (2) is empty or is filled with an organic solvent, in which the produced VOC is soluble.

7. The system according to claim 6, wherein the collection compartment (2) is filled with the organic solvent selected in the group comprising:
- aromatic hydrocarbon compounds, e.g., benzene and toluene,
- alkanes, e.g., dodecane and nonane,
- alkyls,
- alcohols, e.g., methanol,
- esters and ethers,
- ketones, e.g., acetone,
- amines, amides,
- nitrated and halogenated hydrocarbons,
- DMSO,
wherein preferred choices are dodecane or nonane.

8. The system according to claim 7, wherein organic solvents are used alone or in any possible combination of solvents defined in claim 7.

9. The system according to any of the preceding claims, wherein the growth medium is any suitable medium for cultivating microbial strain selected for the procedure of producing VOCs, wherein the preferred media are Luria-Bertani (LB), Terrific Broth (TB), minimal growth medium M9 and similar defined mineral media provided with a suitable amount of agar, which is typically between 0.1 and 3%, preferably between 1 and 3%.

10. The system according to claim 9, wherein the selected growth medium is further supplemented with one or more of the following:
- an inducer, which allows induction of VOCs production in case the microbial strain is provided with an inducible promotor for expression of a gene for VOCs synthesis, wherein examples of a suitable inducer are IPTG, arabinose, tetracycline, rhamnose and similar,
- an appropriate antibiotic or a combination of antibiotics, which allow selection of microbial cells that have successfully received a gene for VOCs synthesis,
- pure carbon source, such as glucose, glycerol, etc. or glucose sources from various sources, said glucose sources being untreated or treated, wherein the glucose source may be hydrolysed waste streams (i.e., glucose juice from cellulose containing waste; ethylene glycol from PET containing waste, etc.),
- other optional components of microbial growth media, such as nitrogen sources and other element sources, enzymes, precursor and/or catalysts.

11. The system according to any of the preceding claims, wherein the system is made from a suitable material that is inert and allows sterilization according to standard microbiological procedures.

12. The method for production of volatile natural compounds using microorganisms grown on solid growth media as the second aspect of the invention, comprises at least the following steps:
a) preparation of the system according to any of the preceding claims and a suitable solid growth medium for inoculation with the selected microbial strain poured into the flask and left to solidify,
b) growth of the selected microbial strain in suitable growth medium,
c) inoculation of the microbial strain prepared in step b) onto the solid growth medium prepared in the container in step a),
d) optionally filling the collection compartment with a suitable organic solvent and optionally with a second liquid separately,
e) sealing of the container using a suitable seal, sealing tape, or any other sealing device,
f) incubation of the container for at least 18 hours, preferably 72 hours, at 20 to 40 °C, preferably at 30 °C,
g) opening of the container, wherein the produced VOC is present in the first vial or the closure of the container.

13. The method according to claim 12, wherein the microbial strain used in the process according to the invention may be any suitable strain known to naturally produce VOC or genetically engineered to produce VOC either spontaneously or following an induction with a suitable inducer.

14. The method according to claim 13, wherein the microbial strain is selected in the group comprising members of genus *Bacillus, Escherichia coli, Saccharomyces cerevisiae,* and fungal strains.

15. Use of the system and/or the method according to any of the preceding claims in production of VOCs, for example limonene, various terpenes, such as g-terpinene, fenchol, a-terpineol, sabinene, (E)-b-ocimene, camphene, b-phellandrene, and terpinolene.
